Europäisches Patentamt

European Patent Office

Office européen des brevets

(19)

(11) Publication number: **0 307 248 B1**

(12) **EUROPEAN PATENT SPECIFICATION**

(45) Date of publication of patent specification: **09.11.94** (51) Int. Cl.⁵: **C12N 15/00**, C12N 5/00, C12P 21/00, C12N 9/64

(21) Application number: **88308387.5**

(22) Date of filing: **12.09.88**

(54) **Method for increasing the expression of polypeptides in recombinant cell culture.**

(30) Priority: **11.09.87 US 97246**

(43) Date of publication of application:
**15.03.89 Bulletin 89/11**

(45) Publication of the grant of the patent:
**09.11.94 Bulletin 94/45**

(84) Designated Contracting States:
**AT BE CH DE ES FR GB IT LI LU NL SE**

(56) References cited:
**EP-A- 0 262 942**
**EP-A- 0 263 908**

**VIROLOGY, vol. 89, no. 2, 1978, pages 570-577; B.A. WOLF and A.R. GOLDBERG: "The expression of Plasminogen activator in Rous Sarcoma virus -transformed cells is controlled both by the virus and the cell"**

(73) Proprietor: **GENENTECH, INC.**
**460 Point San Bruno Boulevard**
**South San Francisco California 94080 (US)**

(72) Inventor: **Levinson, Arthur D.**
**3690 Ralston Avenue**
**Hillsborough Califoria 94010 (US)**
Inventor: **Mather, Jennie P.**
**269 La Prenda**
**Millbrae Califoria 94030 (US)**

(74) Representative: **Armitage, Ian Michael et al**
**MEWBURN ELLIS**
**York House**
**23 Kingsway**
**London WC2B 6HP (GB)**

**Description**

This invention relates to the preparation of heterologous polypeptides in recombinant host cells. In particular, it relates to methods for enhancing the yields of heterologous polypeptide produced in recombinant eukaryotic cell culture.

Many polypeptides have been synthesized in recombinant eukaryotic cell culture, for example, plasminogen activators, inhibin, TGF-$\beta$, activin, prorelaxin, superoxide dismutase, tissue factor, decay accelerating factor, viral antigens and enkephalinase. Of particular interest are the plasminogen activators, especially human tissue type plasminogen activator (t-PA). This enzyme and certain of its amino acid sequence variants are well known and described in the literature, and it is commonly produced in recombinant mammalian cell culture. See for example EP 093,619.

Typically, a eukaryotic host cell such as a yeast, filamentous fungus, vertebrate or invertebrate cell is transfected with nucleic acid encoding a desired polypeptide, transfectants selected for uptake of the nucleic acid, and transfectants identified that are capable of producing the desired polypeptide. Transfectants that produce the largest quantities of polypeptide are identified by selecting subclones that have amplified the nucleic acid encoding the desired polypeptide, such subclones being identified most conveniently by selecting subclones that have coamplified a marker gene such as dihydrofolate reductase (DHFR) through culture in incrementally increasing quantities of a selection agent for the marker gene. Subclones that have amplified the transfected nucleic acid thus are identified by their ability to survive passage into media containing elevated concentrations of selection agent, which in the case of DHFR marker is methotrexate; methotrexate binds to and inactivates DHFR, which establishes a requirement for more DHFR. Amplification of the DHFR gene enables the subclones to grow in the presence of a given level of methotrexate. This technique has facilitated the preparation and identification of recombinants that produce useful quantities of desired polypeptides.

There appear to be limits to the ability of subclone selection as described above to enhance yields of desired polypeptides. Eventually, the cultures grow poorly and contain large proportions of non-viable and aberrant cells. This notwithstanding, the efforts of the art directed at increasing the yields of recombinant polypeptide in general have focused on stimulating the expression levels of polypeptide on a cellular basis, e.g., the use of more powerful promoters and enhancers to increase the polypeptide yield per cell. This strategy ignores the impact of cell viability and growth characteristics on the net yield of polypeptide from the entire cell culture. It would be desirable to increase the cultural as well as the cellular yields of recombinant fermentations. Requirements for capital improvements such as fermenters would be reduced as would operating costs such as media and labor.

Systems are known for increasing cell density in fermentations. These are not considered economic, however, because of increased equipment and media costs. For example, very high cell densities are obtained by fermentations conducted in microtubular devices with continuous or periodic media exchanges, but the fermenters are expensive, recycled with great difficulty, can only be scaled up to a limited extent, and require enormous quantities of fresh media. What is needed is a method for improving the viable cell density in conventional tank fermenters operated in a batch of continuous mode.

Transformed cells exhibit a variety of characteristics, some of which may be possessed by the one cell but not by another. In general, transformed cells are immortalized, i.e., they become capable of infinite cell division, and undergo certain morphological and cultural changes, one of which is the loss of contact inhibition as measured by foci formation in cell monolayers, and another of which is the formation of colonies in soft agar. However, not all immortalized cells will form tumors in nude mice or exhibit loss of contact inhibition or formation of foci in soft agar. Transfection is the transfer of nucleic acid into the permanent genetic repertoire of a host cell. For the purposes of this application transfection and transformation are not to be considered synonymous.

It is well known that transformed mammalian cell lines secrete greater quantities of proteases, including plasminogen activators, than their untransformed progenitors. This appears to be the case notwithstanding whether the transformation occurred as a result of chemical carcinogenesis or viral infection. See M. Duffy et al., "Eur. J. Cancer Clin. Oncol." 20(5):577-82 (1984), R. Isseroff et al., "J. Invest. Dermatol." 80(4): 217-22 (1983), L. Green et al., "Carcinogenesis" 7(3):477-80 (Mar.,1986), B. Rajput et al., "Science" Nov. 8, 1985: p. 672, E. Major et al., "Virology" 136(2):359-67 (1984); J. O'Donnell-Tormey et al. "Cell", 27(1 Pt.2):85-95 (1981); J. Soric et al., "Biochim. Biophys. Acta." 719(3):438-43 (1982); and M. Wainberg et al. "Eur. J. Cancer Clin. Oncol." 18(6):545-51 (1982). In fact, increased secretion of plasminogen activators has been used as one indicium of transformation (E. Gionti et al., "J. Biol. Chem." 258:7190 [1983] and K. Nakamura et al. "Mol. Cell Biol." 2(2):147-53 [1982]). However, L. Skriver et al., "Eur. J. Biochem." 124(2):409-14 (1982) and L. Nielsen et al., "Biochemistry" 21(25):6410-5 (1982) have reported that plasminogen activator

2

can be released from transformants in an enzymatically inactive form, and others observe that not all transformed clones do in fact produce plasminogen activator (E. Angles-Cano et al. "Int. J. Cancer" 33(2)-:277-80 [1984] and C. Cerni, "Oncology" 41(5):349-56 [1984]). The expression of increased amounts of endogenous plasminogen activator has not been conclusively attributed to a particular viral gene or other cellular mechanism, although Sistonen et al. observed that NIH/3T3 cells transfected with the human c-Ha-rasVal 12 oncogene and the neomycin phosphotransferase gene produced foci or colonies (an indicium of transformation) whereas cells expressing abundant c-Ha-ras had lost both their actin and fibronectin networks and showed an increase in plasminogen activator activity (Sistonen et al., "Exp. Cell Res." 168(2)-:518-30 [Feb.,1987].

Oncogenes are nucleotide sequences that confer transformed characteristics on host cells. They typically are derived from viral nucleic acid (v-onc) or cellular DNA (c-onc or protooncogenes). Typically, v-oncs are hybrids of a specific subset of one or more normal, truncated or variant c-onc genes linked to the genomic elements of a retroviral vector that had previously captured or transduced the c-onc gene or its variant. Many oncogenes are known, as shown in the following table from R. Ascione et al. in Papas et al., ed., Gene Amplification and Analysis Vol. 4, page 257 [1986].

Table I

Oncogenes:  Group; Relationships; Origin; and Disease and Activity

| Group Oncogene: Origin [viral(cell)] | Disease | Activity v-onc(c-onc) |
|---|---|---|
| (a$^2$)  src family | | |
| Kinases src  Rous sarcoma virus (chicken) | Sarcoma | Tyrosine Protein Kinase (PK) |
| fps  Fujinami sarcoma virus (chicken) | Sarcoma | Tyrosine PK |
| fes  Feline sarcoma virus (cat) | Sarcoma | Tyrosine PK |
| yes  Yamaguschi sarcoma virus (chicken) | Sarcoma | Tyrosine PK |
| fgr  Gardner-Rasheed virus (cat) | Sarcoma | Tyrosine PK |
| ros  UR2 sarcoma virus (chicken) | Sarcoma | Tyrosine PK |
| fms  McDonough sarcoma virus (cat) | Sarcoma | Tyrosine PK (CSF)/receptor homology) |
| abl  Abelson leukemia virus (mouse) | Leukemia, lymphoma | Tyrosine PK |
| (A$^2$)  Limited sequence homology to src family | | |
| mos  Moloney sarcoma virus (mouse) | Sarcoma | P r o t e i n Kinase d o m a i n homology |
| raf  3611-Murine sarcoma virus (mouse) | Sarcoma | Protein Kinase domain homology |
| mht(mil)$^a$ MH2 avian virus (chicken) | Same as for myc | D o m a i n homology with raf |

4

Table I. (continued)

| Group Oncogene: Origin [viral(cell)] | Disease | Activity v-onc(c-onc) |
|---|---|---|
| erb-B[b]  Erythroblastosis virus | Erythroblastosis and Sarcoma | Domain homology to Tyrosine PK (truncated EGF receptor homology) |
| rel[c]  Reticuloendotheliosis virus | Reticuloendotheliosis | unknown |
| met  (Carcinogen treated human osteosarcoma cell) | unknown (isolated by transfection) | Tyrosine PK |

(B)  ras family

| | | |
|---|---|---|
| Ha-ras  Harvey sarcoma virus (rat) | Sarcoma and erythroleukemia | Protein Kinase (PK) (GTPase binding) |
| Ki-ras  Kirsten sarcoma virus (rat) | Sarcoma and erythroleukemia | PK (GTP binding) |
| N-ras  (Human tumor DNA) | (in vitro 3T3 cell transformation only) | Distinct homology to Ha- and Ki-ras |

Growth Factor and Receptor

(C)  sis family

| | | |
|---|---|---|
| sis  Simian sarcoma virus (wooly monkey) | Sarcoma | Homology to PDGF (gene for PDGF-B) |
| neu  (neuroblastoma isolate rat) | in vitro cell transformation only | Homology to EGF receptor |

Table I. (continued)

| Group Oncogene: Origin [viral(cell)] | Disease | Activity v-onc(c-onc) |
|---|---|---|
| Nuclear | | |
| (D) myc family | | |
| myc MC29 myelocytomatosis virus (chicken) | Sarcoma Carcinoma Myelocytoma | unknown |
| myc[a] MH2 avian virus (chicken) | Sarcoma Carcinoma Myelocytoma | unknown (DNA binding) |
| N-myc (neuroblastoma, retinoblastoma, human) | unknown | unknown (limited homology to c-myc) |
| L-myc (anaplastic lung carcinoma human) | unknown | unknown (very limited homology to c-myc) |
| (E) myb family | | |
| myb Avian myeloblastosis (chicken) | myelo-blastosis | unknown (DNA associated) |
| myb[d] E26 virus (chicken) | myelo-blastosis and erythro-blastosis | unknown |
| (F) Unique family | | |
| fos FBJ osteosarcoma virus (mouse) | osteo-sarcoma | unknown |
| ski SK770 virus (chicken) | squamous carcinoma | unknown |

**Table I.** (continued)

| Group Oncogene: Origin [viral(cell)] | Disease | Activity v-onc(c-onc) |
|---|---|---|
| ets[d] E26 virus (chicken) | myelo-blastosis and erythro-blastosis | unknown |
| erb-A[b] Erythroblastosis virus | erythro-blastosis and sarcoma | Homolog to the glu-cocorticoid receptor |
| bcl 1, bcl 2 | B-cell neo-plasms (isolated by trans-fection) | unknown |
| tcl 1, tcl 2 | T-cell neoplasms | unknown |

[a]Dual oncogenes of MH2 virus.

[b]Dual oncogenes of Avian Erythroblastosis virus.

[c]Very distant homology to src.

[d]Dual oncogenes of tripartite E26 virus genome.

Thus far, oncogenes have been the focus of intense study, at least some of this work presumably having the commercial objective of inactivating or inhibiting oncogenes as a therapeutic approach to the treatment of cancer, or for purposes of designing diagnostic tests. However, oncogenes have found little commercial utility per se.

It is desirable to increase the titer (yield per volume) of polypeptides from recombinant cell cultures, further to increase the yields of polypeptides from recombinant cell culture on a per cell basis,

to enhance the secretion of heterologous polypeptides from recombinant cell culture in batch tank fermentations , and

to increase the viable cell density of recombinant eukaryotic cell cultures.

The European application EP-A-0 262 942 discloses host cell cultures cotransfected with the gene of t-PA and with the RAS gene. This document falls under Article 54(3) EPC, thus it is not relevant to the question of inventive step.

The present invention provides a method comprising (a) transfecting a parent immortalized eukaryotic host cell with nucleic acid encoding a desired polypeptide and with an oncogene; and (b) identifying the transfectants of step (a) that produce greater yields of the desired polypeptide per volume of culture medium than are obtained from the parent host cell transfected with nucleic acid encoding the desired polypeptide but not the oncogene.

In a preferred aspect, the method further comprises culturing the transfectants of step (b) until the desired polypeptide accumulates in the culture and thereafter recovering the desired polypeptide from the culture.

In a preferred embodiment the method comprises (a) transfecting a parent eukaryotic host cell with (i) nucleic acid encoding human tissue plasminogen activator (t-PA) and (ii) an oncogene; and (b) identifying the transfectants of step (a) that produce greater yields of t-PA per volume of culture medium than are obtained from the parent host cell.

The invention also provides a host cell culture comprising (a) an oncogene under the transcriptional control of a heterologous promoter; (b) a gene encoding a desired polypeptide; and (c) culture medium containing the desired polypeptide.

Surprisingly, expression levels of the oncogene do not appear to be correlated with greater yields of the desired polypeptide. A reproducible proportion of transfectants (4/46) with the v-src gene demonstrate enhanced polypeptide expression notwithstanding (1) that the positive clones express extremely minute amounts of v-src protein, or no v-src protein at all as determined by kinase assay, and (2) that several clones failing to demonstrate enhanced polypeptide expression nonetheless produce abundant amounts of v-src protein. High yielding clones do contain DNA encoding the oncogene, however. Thus, we have determined that one should screen transfectants for enhanced polypeptide yields rather than for expression of the oncogene. The elevated t-PA yields produced by the preferred transfectants of this invention secrete copious quantities of heterologous t-PA, and this t-PA is fully active. Also surprising was that the high yielding oncogene transfectants did not acquire any additional detectable transformed characteristics over and beyond the parental cell line, despite the fact that oncogene DNA was detectable in the genome of the transfectants.

Figure 1. Construction of a human preproinsulin expression vector, pSVEHIGDHFR, used to establish an insulin-independent cell line for production of a desired protein.

Figure 2. Construction of a human preproinsulin expression vector, pSVEHIGNeo, used to establish an insulin-independent cell line for production of a desired protein.

Figure 3. Construction of pCVSVD22/preUK54, plasmid used in the construction of pSVEHIGDHFR.

Figure 4. Construction of an ornithine decarboxylase (ODC) expression vector used for amplification of the ODC gene and the cotransfected preproinsulin gene.

A salient feature of this invention is the use of genetically modified host cells to increase viable cell density and total cultural yields of desired polypeptide rather than resorting to expensive fermentation equipment and medium exchanges. This improvement has broken new ground in eukaryotic cell culture since it now is possible to amplify incremental increases in cellular productivity on a culture volume basis. Any oncogene that is capable of realizing this objective is useful herein.

In the present invention, oncogenes are used which, when transfected into a host cell, improve suspension culture saturation density, specific productivity, and suspension culture cell viability when compared to the same parameters exhibited by the host cell prior to transformation with the oncogene. Saturation density is the maximum number of cells per volume of a given culture medium attainable under a given set of culture conditions. Cell viability refers to the percentage of cells at a given saturation density that are viable, i.e. capable of growth and replication. The method of this invention makes it possible to obtain viable cell density increases of about from 1.5 to 10 times the percentage of viable cells otherwise obtainable with the parent host cell cultured under the same conditions, and about from 1.1 to 4 times the saturation culture cell density. Finally, the method of this invention results in increased specific productivity (protein produced per cell) as compared with the parent line. Many oncogenes are known, as set forth above, and others will be discovered in the future. All of them are useful in this invention so long as they confer at least one of these characteristics of improved saturation density, specific productivity, and culture viability, and preferably all of such characteristics.

It is our experience that the host cell should be empirically matched with the oncogene in order to optimize the host cell density and viability. This will be accomplished in typical fashion by providing a host cell that is synthesizing and secreting a desired polypeptide, transfecting the cell with a bank of oncogenes (each selected for example from the various classes described above in Table 1), culturing the cells and analyzing the transfectants for synthesis of enhanced levels of the polypeptide of interest. Any conventional analysis for the polypeptide, e.g., immunological or enzymatic as appropriate, will be used to determine increases in yield of the desired polypeptide. Thus, an oncogene or class of oncogenes that are effective with one host cell may not be as useful with another. The optimal oncogene will be selected by routine screening methods such as the procedure shown in the Examples. For example, v-src and, to a lesser degree, v-myc, are effective in increasing the yields of t-PA from insulin-independent CHO transfectants, but v-fos and activated c-Ha-ras did not demonstrate t-PA yield enhancement under the conditions studied.

Host cells optionally also are transfected with DNA encoding a polypeptide factor that confers growth independence from a substance that otherwise would need to be supplied in the culture medium, i.e., which confers autocrine character on the host cells. Examples of such substances are insulin, proinsulin, fibroblast growth factor, platelet derived growth factor (which also can act as an oncogene), EGF, TGF-$\alpha$, transferrin and receptors, receptor analogues or transducing proteins therefor (some of which, e.g. v-erbB and Her-2,

8

are considered oncogenes). For example, CHO cells, preferred hosts for the production of many polypeptides, are insulin and transferrin dependent but are rendered independent of insulin and transferrin upon transformation with DNA encoding proinsulin and transferrin. The nucleic acid conferring independence from a polypeptide factor is introduced into the host cell before, after or simultaneously with transfection with the oncogene or the nucleic acid encoding the desired polypeptide.

The host cells included within the scope hereof are nucleated cells, ordinarily cells from multicellular organisms and preferably mammalian cells, but also insect, plant or other higher eukaryotic cells. These cells optionally are from a different species than the desired polypeptide to be produced. For example, if the desired polypeptide is human, then the host cell will be derived from a lower animal. However, human cell transfectants are not excluded and in many cases may be preferred. Host cells typically are immortalized, i.e., they are stable cell lines. Suitable host cells include monkey kidney CVI line transformed by SV40 (COS-7, ATCC CRL 1651); human embryonic kidney line (293, Graham, F.L. et al. J. Gen Virol. 36, 59 [1977]); baby hamster kidney cells (BHK, ATCC CCL 10); chinese hamster ovary-cells-DHFR (described by Urlaub and Chasin, PNAS (USA) 77, 4216, [1980]); mouse sertoli cells (TM4, Mather, J.P., Biol. Reprod. 23, 243-251 [1980]); monkey kidney cells (CVI ATCC CCL 70); african green monkey kidney cells (VERO76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); rat hepatoma cells (HTC, M1.54, Baumann, H. et al., J. Cell Biol. 85, 1-8 [1980]); and TR-I cells (Mather, J.P. et al., Annals N.Y. Acad. Sci. 383, 44-68 [1982]).

Expression vectors are used to convey nucleic acid into a host so that it will be expressed by the host. Expression vectors need not be replicable, but this property is convenient for preparing large amounts of the vector. Vectors will contain gene(s) of primary interest (polypeptide factors, desired or product polypeptides, selection markers, oncogenes and the like) under the control of a promoter and with other components necessary for transcription and translation. Vectors generally will contain sequences necessary for the termination of transcription which may affect mRNA expression. These regions are transcribed as polyadenylated segments in the untranslated portion of the mRNA encoding the desired heterologous protein. The 3′ untranslated regions also include transcription termination sites.

Expression vectors contain one or more selection genes, also termed selectable markers. A selection gene encodes a protein, sometimes referred to as a secondary protein, necessary for the survival or growth of a host cell transformed with the vector. Examples of suitable selectable markers for mammalian cells are dihydrofolate reductase (DHFR), thymidine kinase or neomycin. When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR⁻ cells and mouse LTK⁻ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells that were not transformed with the DHFR or TK gene will not be capable of survival in non-supplemented media. Therefore, direct selection of those cells required cell growth in the absence of supplemental nutrients.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1, 327 (1982), mycophenolic acid, Mulligan, R.C. and Berg, P. Science 209, 1422 (1980) or hygromycin, Sugden, B. et al., Mol. Cell. Biol. 5:410-413(1985). The three examples given above employ bacterial genes under eukaryotic control to convey resistance to the appropriate drug neomycin (G418 or geneticin), xgpt (mycophenolic acid) or hygromycin, respectively.

Vectors may contain control regions i.e., specific sequences at the 5′ and 3′ ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription is a CXCAAT region where X may be any nucleotide. At the 3′ end of most eukaryotic genes is an AATAAA sequence which may be the signal for addition of the polyadenylation tail to the 3′ end of the transcribed mRNA.

Vectors will contain promoters, nucleotide segments recognized by RNA polymerase molecules as transcription start signals. Promoters controlling transcription from vectors in mammalian host cells may be obtained from various sources, for example, the genomes of viruses such as: polyoma, Simian Virus 40 (SV40), adenovirus, retroviruses, hepatitis-B virus, and cytomegalovirus, or from heterologous mammalian promoters, e.g. beta actin promoter. The early and late promoters of the SV40 virus are conveniently obtained as an SV40 restriction fragment which also contains the SV40 viral origin of replication. Fiers et al., 1978, "Nature", 273: 113. The immediate early promoter of the human cytomegalovirus is conveniently obtained as a HindIII E restriction fragment. Greenaway, P.J. et al., Gene 18, 355-360 (1982). Of course, promoters from the host cell or related species also are useful herein. Promoters are ligated to the gene, e.g. selection marker, which is to be expressed. The orientation and position of promoters will be known to those skilled in the art.

Vectors optionally will contain an enhancer, a cis-acting element of DNA, usually about from 10-300 bp, that acts on a promoter to increase its transcriptional activity. Transcription of a DNA encoding a desired heterologous protein by higher eukaryotes is increased by inserting an enhancer sequence into the vector. Enhancers are relatively orientation and position independent, having been found 5′ (Laimins, L. et al., PNAS 78, 993 [1981]) and 3′ (Lusky, M.L., et al., Mol. Cell Bio. 3, 1108 [1983]) to the transcription unit, or within an intron (Banerji, J.L. et al., Cell 33, 729 [1983]), or within the coding sequence itself (Osborne, T.F., et al., Mol. Cell Bio. 4, 1293 [1984]). Many enhancer sequences are now known from mammalian genes (globin, elastase, albumin, $\alpha$-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

"Amplification" refers to the increase or replication of an isolated region within a cell's chromosomal DNA. Amplification is achieved using a selection agent e.g. methotrexate (MTX) which inactivates DHFR. Amplification or the making or successive copies of the DHFR gene results in greater amounts of DHFR being produced in the face of greater amounts of MTX. Amplification pressure is applied notwithstanding the presence of endogenous DHFR, by adding ever greater MTX to the media. Amplification of a desired gene can be achieved by cotransfecting a mammalian host cell with a plasmid having a DNA encoding a desired protein and the DHFR or amplification gene so that cointegration can occur. One ensures that the cell requires more DHFR, which requirement is met by replication of the selection gene, by selecting only for cells that can grow in successive rounds of ever-greater MTX concentration. So long as the gene encoding a desired protein has cointegrated with the amplifiable gene, replication of this gene gives rise to replication of the gene encoding the desired protein. The result is that increased copies of the gene, i.e. an amplified gene, encoding the desired protein express more of the desired protein.

"Transfection" refers to the taking up of an expression vector by a host cell whether or not any coding sequences are in fact expressed. Numerous methods of transfection are known to the ordinarily skilled artisan, for example, $CaPO_4$ and electroporation. Successful transfection is generally recognized when any indication of the operation of this vector occurs within the host cell. However, in the context of the present invention transfection ordinarily refers to stable integration of an oncogene into a host culture over numerous generations.

The desired polypeptides include polypeptides having as few as about 5 residues, but more typically are proteins. The desired polypeptides are from any species of plant, microbe or animal, or they may be variants or synthetic polypeptides having no counterpart in nature. Ordinarily, the polypeptides will be animal, preferably human, proteins having therapeutic utility. Enzymes and hormones are the most prevalent classes of desired polypeptides. For example, such polypeptides include inhibin, plasminogen activators, TGF-$\beta$, activin, prorelaxin, superoxide dismutases, tissue factor, decay accelerating factor, viral antigens or enkephalinase. The preferred polypeptide is human t-PA.

The host vector systems constructed in accordance with this invention are cultured under conditions and in media otherwise appropriate for the host cell in question. It will be understood that each host-vector system will require a different medium for optimal performance, but that the selection of the best culture medium will be within the ordinary skill. Similarly, other culture parameters such as temperature, aeration rate and gas content, and the like will be a matter for routine experimentation.

Viable cells are determined by any conventional method, although the preferred technique is trypan blue staining followed by hemocytometer analysis. In general, viable cell analysis is made when saturation density in the culture has been reached, in the case of CHO cells after about 7 days of culture. It is during the saturation phase that the impact of the oncogene is greatest, both in terms of the maximum density reached as well as the proportion of healthy viable cells in the saturated culture.

In order to simplify the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences, restriction sites, in the DNA. The various restriction enzymes used herein are commercially available and their reaction conditions, cofactors and other requirements were used as would be known to the ordinarily skilled artisan. For analytical purposes, typically 1 $\mu$g of plasmid or DNA fragment is used with about 2 units of enzyme in about 20 $\mu$l of buffer solution. For the purpose of isolating DNA fragments for plasmid construction, typically 5 to 10 $\mu$g of DNA would be digested with 20 to 40 units of enzyme in a larger volume. Appropriate buffers and substrate amounts for particular restriction enzymes are specified by the manufacturer. Incubation times of about one hour at 37°C are ordinarily used, but may vary in accordance with the supplier's instructions. After digestion the reaction was run directly on a gel to isolate the desired fragment.

"Dephosphorylation" refers to the removal of the terminal 5′ phosphates by treatment with bacterial alkaline phosphatase (BAP). This procedure prevents the two restriction cleaved ends of a DNA fragment from "circularizing" or forming a closed loop that would impede insertion of another DNA fragment at the restriction site. Procedures and reagents for dephosphorylation are conventional. Maniatis, T. et al., CSHL 1982, Molecular Cloning pp. 133-134. Reactions using BAP are carried out in 50mM Tris at 68°C to suppress the activity of any exonucleases which may be present in the enzyme preparations. Reactions were run for one hour. Following the rection the DNA fragment is gel purified.

"Oligonucleotides" refers to short length single or double stranded polydeoxynucleotides which are chemically synthesized by known methods and then purified on polyacrylamide gels.

"Ligation" refers to the process of forming phosphodiester bonds between two double stranded nucleic acid fragments (Maniatis, T. et al., Id., p. 146). Unless otherwise provided, ligation may be accomplished using known buffers and conditions with 10 units of T4 DNA ligase ("ligase") per 0.5 $\mu$g of approximately equimolar amounts of the DNA fragments to be ligated.

"Filling" or "blunting" refers to the procedures by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15$\mu$g of the target DNA in 10mM $MgCl_2$, 1mM dithiothreitol, 50mM NaCl, 10mM Tris (pH 7.5) buffer at about 37°C in the presence of 8 units of the Klenow fragment of DNA polymerase I and 250 $\mu$M of each of the four deoxynucleoside triphosphates. The incubation generally is terminated after 30 min. phenol and chloroform extraction and ethanol precipitation.

"Northern" blotting is a method by which the presence of a cellular mRNA is confirmed by hybridization to a known, labelled oligonucleotide or DNA fragment. For the purposes herein, unless otherwise provided, Northern analysis shall mean electrophoretic separation of the mRNA on 1 percent agarose in the presence of a denaturant (formaldehyde 7%), and transfer to nitrocellulose hybridization to the labelled fragment as described by Maniatis, T. et al., Id., p. 202.

The following examples are only intended to illustrate the invention and are not to be considered limiting thereof.

Example 1

Construction of Insulin Autocrine Cell Line C2B13

A) Construction of Human Proinsulin Expression Vector

The cDNA clone of the insulin gene, pHI3, provided the coding sequence of the human preproinsulin gene for construction of plasmids to direct the expression of preproinsulin in transfected mammalian cells. The vector pSVEHIGDHFR containing the SV40 promoter, the cDNA encoding human preproinsulin, the hepatitis B virus surface antigen polyadenylation site and the cDNA encoding mouse dihydrofolate reductase was constructed.

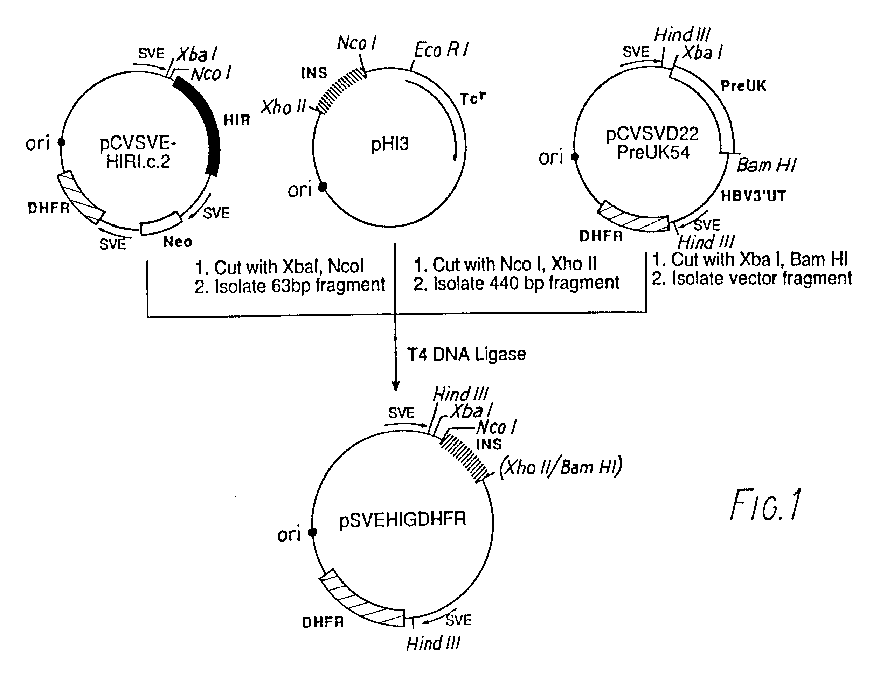

Figure 1 shows the steps for construction of the preproinsulin expression vector used to establish an insulin independent host cell line. The three parts of the construction of pSVEHIG are detailed below:

a) pSVEHIGDHFR

1) The cDNA encoding human preproinsulin was obtained in a 440bp fragment from pHI3 by a (NcoI - XhoII) digest. pHI3 is described in Sures, I. et al., Science 208:57 (1980). The 440 bp fragment containing the cDNA encoding preproinsulin was isolated.

2) A 63bp XbaI-NcoI fragment was isolated from the 5′ end of the insulin receptor plasmid (pCVSVE-HIRIc.2, European Publication No. 0192392, published August 27, 1986). This fragment functioned as a linker-adapter to fuse the 5′ end of the cDNA encoding preproinsulin to the SV40 early promoter.

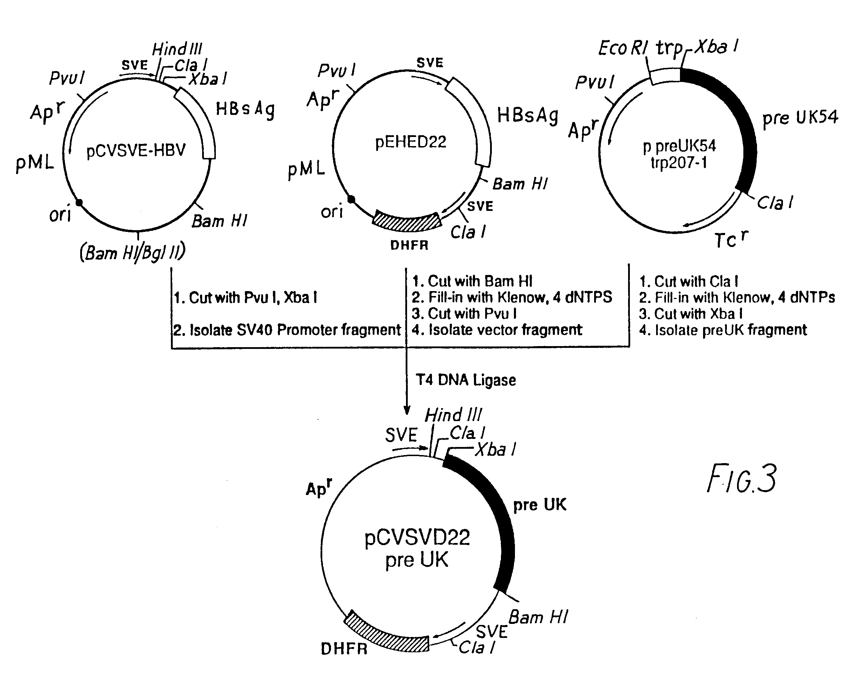

3) The vector, pCVSVD22/preUK54, providing the plasmid backbone which is ligated to the 63bp linker and proinsulin gene coding sequences, was prepared as described below. pCVSVD22/preUK54, the plasmid backbone, is the product of a three fragment ligation as diagramed in Figure 3.

i) The SV40 early promoter is obtained by digesting plasmid pCVSVEHBV (European Patent Application Publication No. 0117060, published August 29, 1984) with PvuI and XbaI.

ii) The fragment containing the preurokinase cDNA was obtained from plasmid p preUK54 trp207-I (European Patent Application Publication No. 0092182, published October 26, 1983). The plasmid was digested with ClaI. The ClaI ends are made blunt by a filling reaction. The Klenow fragment of DNA polymerase I plus all 4 deoxyribonucleotide triphosphates are added to fill in the ClaI protruding single stranded ends. After the fill-in, plasmid DNA is digested with the second enzyme, XbaI. The XbaI-ClaI (filled) preUK54 cDNA fragment was then isolated.

iii) The vector fragment containing the bacterial origin of replication, the DHFR cDNA, eukaryotic expression unit, and the 3′ untranslated region of hepatitis virus surface antigen was derived from pEHED22 (U.S. Patent No. 4,624,918, issued November 25, 1986). The plasmid was first cut with BamHI. The protruding BamHI ends were then blunted by a filling reaction with Klenow DNA polymerase I as in the procedure detailed for ClaI blunting described above. Following the BamHI digestion and fill-in, the DNA was cut with XbaI and the large 4.3 Kb fragment isolated.

These three fragments were mixed together and ligated in a three fragment, concerted ligation. The recombinant pCVSVD22/preUK54 was recovered. Ligation of a filled ClaI site to a filled BamHI site results in an intact BamHI site at this junction.

To construct pSVEHIGDHFR, pCVSVD22/preUK54 was digested with XbaI and BamHI and the vector fragment isolated.

The final three part ligation to yield pSVEHIGDHFR used: a) the 440bp NcoI-XhoII fragment containing the cDNA for proinsulin; b) a 63bp Xba1-NcoI fragment from pCVSVE-HIRc-2 to link the cDNA to the SV40 early promoter; and, c) the XbaI-BamHI vector fragment from pCVSVD22/ preUK54 containing the SV40-DHFR transcription unit, the ampicillin resistance marker origin of replication in E. coli, the hepatitis surface antigen 3′ end with the polyadenylation and transcription termination site. The three fragments were ligated together in a concerted three-way ligation and transformed into E.coli. Transformants were analyzed and the desired recombinant was identified.

b) pSVEHIGNeo

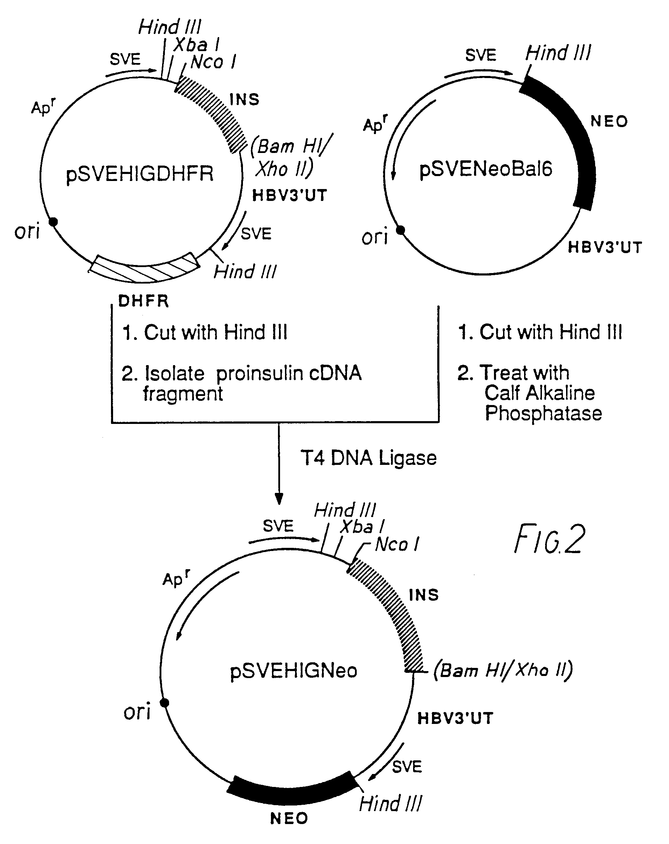

Figure 2 shows the steps for construction of the preproinsulin expression vector pSVEHIGNeo.

This vector was constructed via a two fragment construction. The first fragment was a HindIII fragment from pSVEHIGDHFR described above. Included in the fragment was the cDNA encoding preproinsulin and the SV40 early promoter that initiates transcription of the DNA encoding DHFR. The plasmid backbone comprising the second fragment was obtained by digestion at the unique HindIII site just downstream of the SV40 promoter of pSVENEOBa16 (European Publication No.0160457, published November 6, 1985). The linearized plasmid was then treated with calf alkaline phosphatase to prevent recircularization. The HindIII fragment from pSVEHIGDHFR was inserted at the unique HindIII site of pSVENeoBa16 such that the SV40 promoter originally transcribing the mouse SV40-DHFR transcription unit is upstream of the preproinsulin gene. After ligation the plasmid is transformed into E. coli 294 cells.

Recombinant cells are identified by restriction analysis to insure proper orientation of the fragment containing the proinsulin cDNA. In the proper orientation the SV40 promoter that originally transcribed the bacterial Neo gene is now upstream and initiates transcription of the preproinsulin cDNA.

c) pEO

A vector containing the ornithine decarboxylase (ODC) cDNA under control of the SV40 promoter, having a hepatitis B polyadenylation sequence and an ampicillin gene for selection in E. coli, was constructed. The endogenous ODC gene can be amplified in mammalian cells by selection with the ODC inhibitor, alpha difluoromethylornithine (DFMO). (McConlogue, L. & Coffino, P., J. Biol. Chem. 258,8384-8388 [1983]; McConlogue, L. & Coffino, P., J. Biol. Chem. 258:12083-12086 [1983]).

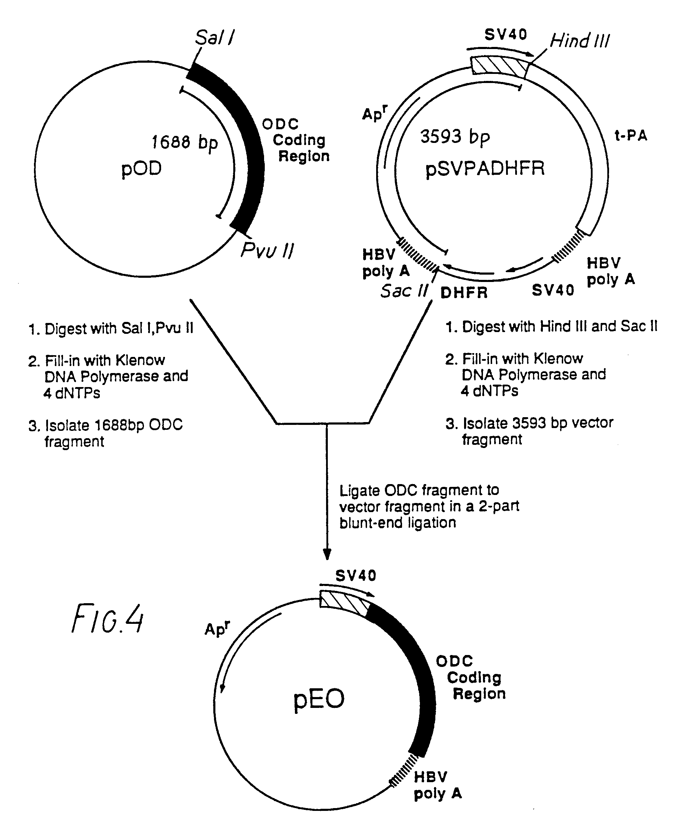

Figure 4 shows the steps for construction of pEO via a two-fragment ligation.

1. A 1688 bp ODC fragment containing the entire coding region of ODC was obtained from a plasmid containing ODC cDNA cloned into pBR322 (McConlogue, L. et al. Proc. Natl. Acad. Sci. USA 81:540-544 [1984]; Gupta, M & Coffino, P. J. Biol. Chem. 260:2941-2944 [1985]). The plasmid was cut with SalI and PvuII. The ends were blunted by filling in with Klenow, and the 1688 pair ODC fragment was isolated on a gel.

2. A 3593 bp fragment containing the SV40 early promoter, the hepatitis polyadenylation sequence, and the AMP gene for selection in E. coli was isolated from plasmid pSVPADHFR. (European Patent Application Publication No. 0,093,619, referred to therein as pETPFR which was modified by the addition of 192bp fragment at the SV40 promoter 5′ to the DNA encoding t-PA. The additional 192 bp fragment included an extra HindIII site.) The plasmid was cut with HindIII and SacII and the ends were filled in with Klenow DNA polymerase and the 3593 fragment was isolated on a gel.

These two fragments were then ligated together in a two-part ligation to form pEO. (See Figure 4). The orientation and configuration of the fragments in the final plasmid was checked by restriction analysis.

## B) Selection of t-PA Insulin-Independent Cells (C2B13)

In order to determine whether endogenously produced proinsulin would be sufficient to support the secretion of a desired protein (e.g. tPA) in an insulin-independent fashion, a transfection was performed in a manner similar to that described in example 2, but using a host cell previously transformed to express a desired protein, in this case tPA. The vector, pSVEHIGNeo, described in example 1 was transfected into the CHO cell line containing amplified tPA and DHFR (referred to as C2B) (European Publication No. 0093619). Transfection was by the calcium-phosphate coprecipitation method. (Simonsen, C.C. & Levinson, A.D., PNAS 80:2495-2499 [1983]; Wigler, M. et al., PNAS [USA]-76:1242-1255 [1979]). Transfected cells expressing the Neo gene were selected by growth in medium containing G418.

The C2B preproinsulin transfected cells were selected for insulin independence in serum-free, insulin-free (SFIF) spinners and plates. The serum-free medium was standard 350mOsm insulin-free F-12/DME medium described above: glucose; 2xGHT; 10mM Hepes; 1.0Mg/L transferrin; 1x trace elements; $1\mu$M linoleic; $1\times10^{-10}$ M $T_3$ and $1\times10^{-8}$ M hydrocortisone, estradiol and progesterone.

After nearly two weeks of selection for insulin-independence, surviving cells were rescued from both the plates and the spinners with medium containing 5% dialyzed, extracted FBS, and 23 clones were derived by limiting dilution. These clones were screened for tPA production under serum-free conditions in the absence of insulin and in the presence of varying insulin concentrations (including the optimal concentration of 20 $\mu$g/ml insulin). Clone 13 was picked as the most promising for further work.

C2B (control) cells, C2B/clone 13 insulin-independent cells and the 100 $\mu$M DFMO amplified pool were rinsed three times in SFIF medium and resuspended in SFIF medium. Clone 13 and the 100 $\mu$M insulin-independent cell lines produced tPA in the absence of insulin at titers equivalent to those achieved by the C2B control cell line in the presence of optimal concentrations of insulin.

## Example 2

### Preparation and Indentification of t-PA Hypersecreting Clones

Unless otherwise stated, the culture medium was 50/50 F-12/DMEM GHT⁻ medium containing 7.5% dialyzed fetal bovine serum. It will be understood that other culture media will produce greater quantities of t-PA and produce higher cell densities than are obtained with this medium, but serum-containing media resulted in somewhat improved cell viability. About 500,000 C2B13 cells cultured on extracellular matrix plates (Accurate Chemical and Scientific Co.) were transfected with a $CaPO_4$ precipitate of pSV5GPT (Mulligan et al. "Proc. Natl. Acad. Sci. U.S.A." 78:2072-2076 [1981]) and p158 SVE src LTR (Snydor et al. "Cell" 32:891-901 [1983]) (1:10 by weight) at 37°C for 3 hours. Control plasmid (pBR322) was transfected under the same conditions. The cells were then cultured at 37°C under 5% $CO_2$ for 2 weeks on culture medium containing 200-400 micrograms of hygromycin B/ml. The medium was exchanged every 2 days during this period in order to maintain the antibiotic level. Thereafter, colonies which had developed on the plates were picked and analyzed for t-PA synthesis and viability analysis. Each clone was cultured by seeding 200,000 cells into culture dishes containing 0.5 ml of culture medium and assayed on day 7 and 11 by t-PA ELISA for t-PA expression levels. After day 6 and for every 2 days thereafter the volume per dish was brought up to 0.5 ml by adding more culture medium after each 30λ sample was withdrawn for assay.

This procedure was repeated four times, resulting in 4 positive clones out of about 46 transfectant clones. A positive clone was defined as one in which the amount of t-PA was at least 50% greater than the parental line on day 6. One clone, 2C2, was selected for further analysis.

Clone 2C2 and C2B13 were cultured as described above except that t-PA analyses were made every 2 days. The results are shown below as a percentage increase in t-PA yield for clone 2C2 over the yields obtained with C2B13 cultured under the same conditions.

| Day | | | | | | |
|---|---|---|---|---|---|---|
| Day | 4 | 6 | 8 | 10 | 12 | 14 |
| Yield Increase (%) | 64 | 60 | 32 | 56 | 56 | 68 |

The amount of t-PA produced during the first day of cultivation by 2C2 cells has ranged up to about twice that of the parent cell line in repeat experiments, thus indicating that the amount of t-PA per cell is increased by the src transformation. The enhanced culture yields also were attributable to increased cell culture density. In fermenter runs the 2C2 cell line achieved densities of about 5-7 X $10^6$ cells/ml., whereas the parental line typically could only reach densities of 1-3 X $10^6$ cells/ml.

The src transfectants also exhibited a higher proportion of viable cells than cultures of the parent line. Trypan blue staining (Gibco) was conducted by trypsinizing the cells in the culture wells, removing the fluid from the well, centrifuging the suspension to obtain a cell pellet, retrypsinizing the pellet, suspending the disaggregated cells in culture medium, counting the total cells by Coulter counter, staining with trypan blue and counting dead (blue) cells on a hemocytometer. The percentage of viable cells on days 7 and 8 of culturing were 15% and 13% respectively for C2B13 (the parental) cells and 92% and 98% respectively for 2C2 cells (the src transfectants), thus demonstrating the ability of the src transfection to confer long-term, high density cultural stability on the host cells.

Example 3

t-PA Expression Enhancement in Non-autocrine Cells

Example 2 was repeated except that the transformed cell was the t-PA transformed CHO cell line C2B described in Example 1. The C2B stable transformant is insulin dependent. 1 out of about 20 src transformants exhibited significantly enhanced t-PA yields as judged by the criteria of Example 2.

**Claims**
**Claims for the following Contracting States : AT, BE, CH, DE, FR, GB, IT, LI, LU, NL, SE**

**1.** A method comprising:
(a) transfecting a parent immortalized eukaryotic host cell with nucleic acid encoding a desired polypeptide and with an oncogene; and
(b) identifying the transfectants of step (a) that produce greater yields of the desired polypeptide per volume of culture medium than are obtained from the parent host cell transfected with nucleic acid encoding the desired polypeptide but not the oncogene.

**2.** The method of claim 1 wherein the parent host cell is transfected with the nucleic acid encoding the desired polypeptide prior to transfecting the host cell with the oncogene.

**3.** The method of claim 1 or 2 wherein the eukaryotic host cell is a mammalian cell.

**4.** The method of claim 1, 2 or 3 wherein the polypeptide is human.

5. The method of any one of claims 1-4 wherein the polypeptide is a plasminogen activator, inhibin, TGF-β, activin, prorelaxin, superoxide dismutase, tissue factor, decay accelerating factor, a viral antigen, or an enkephalinase.

6. The method of claim 5 wherein the polypeptide is t-PA.

7. The method of any one of claims 1-6 wherein the host cell also is transfected with an amplifiable marker.

8. The method of any one of claims 1-7 wherein the oncogene is a viral nucleic acid.

9. The method of claim 8 wherein the oncogene is v-src.

10. The method of any one of claims 1-9 wherein the polypeptide is not required for the viability or growth of the parent host cell.

11. The method of any one of claims 1-10 further comprising culturing the transfectants of step b) until the desired polypeptide accumulates in the culture and thereafter recovering the desired polypeptide from the culture.

12. The method of any one of claims 1-11 wherein the polypeptide is a secreted polypeptide.

13. The method of any one of claims 1-12 wherein the host cell is also transfected with nucleic acid encoding a polypeptide growth factor.

14. The method of claim 13 wherein the growth factor is insulin or proinsulin.

15. The method of any one of claims 1-14 wherein the nucleic acid encoding the desired polypeptide is not under the transcriptional control of its native cellular transcriptional control sequences.

16. The method of claim 15 wherein the nucleic acid encoding the desired polypeptide is under the control of a viral promoter.

17. The method of claim 1 wherein the polypeptide is human tissue plasminogen activator (t-PA).

18. The method of claim 17 wherein the oncogene is v-src and the host cell is of a non-primate mammalian origin.

19. The method of claim 17 or 18 wherein the host cell also is transfected with nucleic acid encoding a polypeptide growth factor.

20. A host cell culture comprising
    (a) an oncogene under the transcriptional control of a heterologous promoter;
    (b) a gene encoding a desired polypeptide; and
    (c) culture medium containing the desired polypeptide.

21. The cell culture of claim 20 wherein the desired polypeptide is not required for the growth or survival of the cell culture.

**Claims for the following Contracting State : ES**

1. A method comprising:
    (a) transfecting a parent immortalized eukaryotic host cell with nucleic acid encoding a desired polypeptide and with an oncogene; and
    (b) identifying the transfectants of step (a) that produce greater yields of the desired polypeptide per volume of culture medium than are obtained from the parent host cell transfected with nucleic acid encoding the desired polypeptide but not the oncogene.

2. The method of claim 1 wherein the parent host cell is transfected with the nucleic acid encoding the desired polypeptide prior to transfecting the host cell with the oncogene.

3. The method of claim 1 or 2 wherein the eukaryotic host cell is a mammalian cell.

4. The method of claim 1, 2 or 3 wherein the polypeptide is human.

5. The method of any one of claims 1-4 wherein the polypeptide is a plasminogen activator, inhibin, TGF-$\beta$, activin, prorelaxin, superoxide dismutase, tissue factor, decay accelerating factor, a viral antigen, or an enkephalinase.

6. The method of claim 5 wherein the polypeptide is t-PA.

7. The method of any one of claims 1-6 wherein the host cell also is transfected with an amplifiable marker.

8. The method of any one of claims 1-7 wherein the oncogene is a viral nucleic acid.

9. The method of claim 8 wherein the oncogene is v-src.

10. The method of any one of claims 1-9 wherein the polypeptide is not required for the viability or growth of the parent host cell.

11. The method of any one of claims 1-10 further comprising culturing the transfectants of step b) until the desired polypeptide accumulates in the culture and thereafter recovering the desired polypeptide from the culture.

12. The method of any one of claims 1-11 wherein the polypeptide is a secreted polypeptide.

13. The method of any one of claims 1-12 wherein the host cell is also transfected with nucleic acid encoding a polypeptide growth factor.

14. The method of claim 13 wherein the growth factor is insulin or proinsulin.

15. The method of any one of claims 1-14 wherein the nucleic acid encoding the desired polypeptide is not under the transcriptional control of its native cellular transcriptional control sequences.

16. The method of claim 15 wherein the nucleic acid encoding the desired polypeptide is under the control of a viral promoter.

17. The method of claim 1 wherein the polypeptide is human tissue plasminogen activator (t-PA).

18. The method of claim 17 wherein the oncogene is v-src and the host cell is of a non-primate mammalian origin.

19. The method of claim 17 or 18 wherein the host cell also is transfected with nucleic acid encoding a polypeptide growth factor.

**Patentansprüche**

**Patentansprüche für folgende Vertragsstaaten : AT, BE, CH, DE, FR, GB, IT, LI, LU, NL, SE**

1. Verfahren umfassend:
   (a) das Transfizieren einer immortalisierten eukaryotischen Wirtsmutterzelle mit Nukleinsäure, die für ein erwünschtes Polypeptid kodiert, und mit einem Onkogen; und
   (b) das Identifzieren der Transfektanten von Schritt (a), die größere Ausbeuten des erwünschten Polypeptids pro Volumen Kulturmedium erzeugen, als aus der Wirtsmutterzelle, die mit Nukleinsäure, die für das erwünschte Polypeptid kodiert, jedoch nicht mit dem Onkogen transfiziert ist, gewonnen werden.

**2.** Verfahren nach Anspruch 1, worin die Wirtsmutterzelle mit der für das erwünschte Polypeptid kodierenden Nukleinsäure vor dem Transfizieren der Wirtszelle mit dem Onkogen transfiziert wird.

**3.** Verfahren nach Anspruch 1 oder 2, worin die eukaryotische Wirtszelle eine Säugetierzelle ist.

**4.** Verfahren nach Anspruch 1, 2 oder 3, worin das Polypeptid menschlich ist.

**5.** Verfahren nach einem der Ansprüche 1-4, worin das Polypeptid ein Plasminogenaktivator, Inhibin, TGF-$\beta$, Activin, Prorelaxin, Superoxiddismutase, Gewebefaktor, abbaubeschleunigender Faktor, ein virales Antigen oder eine Enkephalinase ist.

**6.** Verfahren nach Anspruch 5, worin das Polypeptid t-PA ist.

**7.** Verfahren nach einem der Ansprüche 1-6, worin die Wirtszelle auch mit einem verstärkbaren Marker transfiziert wird.

**8.** Verfahren nach einem der Ansprüche 1-7, worin das Onkogen eine virale Nukleinsäure ist.

**9.** Verfahren nach Anspruch 8, worin das Onkogen v-src ist.

**10.** Verfahren nach einem der Ansprüche 1-9, worin das Polypeptid für die Lebensfähigkeit oder das Wachstum der Wirtsmutterzelle nicht erforderlich ist.

**11.** Verfahren nach einem der Ansprüche 1-10, weiters umfassend das Kultivieren der Transfektanten von Schritt (b), bis sich das erwünschte Polypeptid in der Kultur ansammelt und das anschließende Gewinnen der erwünschten Polypeptids aus der Kultur.

**12.** Verfahren nach einem der Ansprüche 1-11, worin das Polypeptid ein sekretiertes Polypeptid ist.

**13.** Verfahren nach einem der Ansprüche 1-12, worin die Wirtszelle auch mit Nukleinsäure transfiziert wird, die für einen Polypeptid-Wachstumsfaktor kodiert.

**14.** Verfahren nach Anspruch 13, worin der Wachstumsfaktor Insulin oder Proinsulin ist.

**15.** Verfahren nach einem der Ansprüche 1-14, worin die für das erwünschte Polypeptid kodierende Nukleinsäure nicht unter der transkriptionalen Steuerung ihrer nativen zellularen transkriptionalen Steuersequenzen steht.

**16.** Verfahren nach Anspruch 15, worin die für das erwünschte Polypeptid kodierende Nukleinsäure unter der Steuerung eines viralen Promotors steht.

**17.** Verfahren nach Anspruch 1, worin das Polypeptid menschlicher Gewebeplasminogenaktivator (t-PA) ist.

**18.** Verfahren nach Anspruch 17, worin das Onkogen v-src ist und die Wirtszelle einen Nichtprimaten-Säugetierursprung aufweist.

**19.** Verfahren nach Anspruch 17 oder 18, worin die Wirtszelle auch mit Nukleinsäure transfiziert wird, die für einen Polypeptid-Wachstumsfaktor kodiert.

**20.** Wirtszellenkultur umfassend
    (a) ein Onkogen unter der transkriptionalen Steuerung eines heterologen Promotors;
    (b) ein für ein erwünschtes Polypeptid kodierendes Gen; und
    (c) ein das erwünschte Polypeptid enthaltendes Kulturmedium.

**21.** Zellkultur nach Anspruch 20, worin das erwünschte Polypeptid für das Wachstum oder Überleben der Zellkultur nicht erforderlich ist.

**Patentansprüche für folgenden Vertragsstaat : ES**

1. Verfahren umfassend:
   (a) das Transfizieren einer immortalisierten eukaryotischen Wirtsmutterzelle mit Nukleinsäure, die für ein erwünschtes Polypeptid kodiert, und mit einem Onkogen; und
   (b) das Identifzieren der Transfektanten von Schritt (a), die größere Ausbeuten des erwünschten Polypeptids pro Volumen Kulturmedium erzeugen, als aus der Wirtsmutterzelle, die mit Nukleinsäure, die für das erwünschte Polypeptid kodiert, jedoch nicht mit dem Onkogen transfiziert ist, gewonnen werden.

2. Verfahren nach Anspruch 1, worin die Wirtsmutterzelle mit der für das erwünschte Polypeptid kodierenden Nukleinsäure vor dem Transfizieren der Wirtszelle mit dem Onkogen transfiziert wird.

3. Verfahren nach Anspruch 1 oder 2, worin die eukaryotische Wirtszelle eine Säugetierzelle ist.

4. Verfahren nach Anspruch 1, 2 oder 3, worin das Polypeptid menschlich ist.

5. Verfahren nach einem der Ansprüche 1-4, worin das Polypeptid ein Plasminogenaktivator, Inhibin, TGF-β, Activin, Prorelaxin, Superoxiddismutase, Gewebefaktor, abbaubeschleunigender Faktor, ein virales Antigen oder eine Enkephalinase ist.

6. Verfahren nach Anspruch 5, worin das Polypeptid t-PA ist.

7. Verfahren nach einem der Ansprüche 1-6, worin die Wirtszelle auch mit einem verstärkbaren Marker transfiziert wird.

8. Verfahren nach einem der Ansprüche 1-7, worin das Onkogen eine virale Nukleinsäure ist.

9. Verfahren nach Anspruch 8, worin das Onkogen v-src ist.

10. Verfahren nach einem der Ansprüche 1-9, worin das Polypeptid für die Lebensfähigkeit oder das Wachstum der Wirtsmutterzelle nicht erforderlich ist.

11. Verfahren nach einem der Ansprüche 1-10, weiters umfassend das Kultivieren der Transfektanten von Schritt (b), bis sich das erwünschte Polypeptid in der Kultur ansammelt und das anschließende Gewinnen der erwünschten Polypeptids aus der Kultur.

12. Verfahren nach einem der Ansprüche 1-11, worin das Polypeptid ein sekretiertes Polypeptid ist.

13. Verfahren nach einem der Ansprüche 1-12, worin die Wirtszelle auch mit Nukleinsäure transfiziert wird, die für einen Polypeptid-Wachstumsfaktor kodiert.

14. Verfahren nach Anspruch 13, worin der Wachstumsfaktor Insulin oder Proinsulin ist.

15. Verfahren nach einem der Ansprüche 1-14, worin die für das erwünschte Polypeptid kodierende Nukleinsäure nicht unter der transkriptionalen Steuerung ihrer nativen zellularen transkriptionalen Steuersequenzen steht.

16. Verfahren nach Anspruch 15, worin die für das erwünschte Polypeptid kodierende Nukleinsäure unter der Steuerung eines viralen Promotors steht.

17. Verfahren nach Anspruch 1, worin das Polypeptid menschlicher Gewebeplasminogenaktivator (t-PA) ist.

18. Verfahren nach Anspruch 17, worin das Onkogen v-src ist und die Wirtszelle einen Nichtprimaten-Säugetierursprung aufweist.

19. Verfahren nach Anspruch 17 oder 18, worin die Wirtszelle auch mit Nukleinsäure transfiziert wird, die für einen Polypeptid-Wachstumsfaktor kodiert.

**Revendications**
**Revendications pour les Etats contractants suivants : AT, BE, CH, DE, FR, GB, IT, LI, LU, NL, SE**

1. Procédé comprenant :
   (a) la transfection d'une cellule-hôte eucaryotique mère, rendue immortelle, avec un acide nucléique codant pour un polypeptide désiré et avec un oncogène ; et
   (b) l'identification des transfectants de l'étape (a) qui produisent des quantités du polypeptide désiré par volume de milieu de culture qui sont supérieures à celles obtenues à partir de la cellule-hôte mère transfectée avec un acide nucléique codant pour le polypeptide désiré mais non avec l'oncogène.

2. Procédé suivant la revendication 1, dans lequel la cellule-hôte mère est transfectée avec l'acide nucléique codant pour le polypeptide désiré avant transfection de la cellule-hôte avec l'oncogène.

3. Procédé suivant la revendication 1 ou 2, dans lequel la cellule-hôte eucaryotique est une cellule de mammifère.

4. Procédé suivant la revendication 1, 2 ou 3, dans lequel le polypeptide est d'origine humaine.

5. Procédé suivant l'une quelconque des revendications 1 à 4, dans lequel le polypeptide est un activateur du plasminogène, l'inhibine, le TGF-$\beta$, l'activine, la prorelaxine, la superoxyde-dismutase, un facteur tissulaire, le facteur d'accélération de dégradation, un antigène viral ou une enképhalinase.

6. Procédé suivant la revendication 5, dans lequel le polypeptide est le t-PA.

7. Procédé suivant l'une quelconque des revendications 1 à 6, dans lequel la cellule-hôte est également transfectée avec un marqueur amplifiable.

8. Procédé suivant l'une quelconque des revendications 1 à 7, dans lequel l'oncogène est un acide nucléique viral.

9. Procédé suivant la revendication 8, dans lequel l'oncogène est l'oncogène v-src.

10. Procédé suivant l'une quelconque des revendications 1 à 9, dans lequel le polypeptide n'est pas nécessaire à la viabilité ou la croissance de la cellule-hôte mère.

11. Procédé suivant l'une quelconque des revendications 1 à 10, comprenant en outre la culture des transfectants de l'étape (b) jusqu'à ce que le polypeptide désiré s'accumule dans la culture, puis la séparation du polypeptide désiré de la culture.

12. Procédé suivant l'une quelconque des revendications 1 à 11, dans lequel le polypeptide est un polypeptide sécrété.

13. Procédé suivant l'une quelconque des revendications 1 à 12, dans lequel la cellule-hôte est également transfectée avec un acide nucléique codant pour un facteur de croissance polypeptidique.

14. Procédé suivant la revendication 13, dans lequel le facteur de croissance est l'insuline ou la proinsuline.

15. Procédé suivant l'une quelconque des revendications 1 à 14, dans lequel l'acide nucléique codant pour le polypeptide désiré n'est pas sous le contrôle transcriptionnel de ses séquences cellulaires naturelles de régulation de transcription.

16. Procédé suivant la revendication 15, dans lequel l'acide nucléique codant pour le polypeptide désiré est sous le contrôle d'un promoteur viral.

17. Procédé suivant la revendication 1, dans lequel le polypeptide est l'activateur du plasminogène tissulaire humain (t-PA).

EP 0 307 248 B1

**18.** Procédé suivant la revendication 17, dans lequel l'oncogène est l'oncogène v-src et la cellule-hôte est issue d'un mammifère ne consistant pas en un primate.

**19.** Procédé suivant la revendication 17 ou 18, dans lequel la cellule-hôte est également transfectée avec un acide nucléique codant pour un facteur de croissance polypeptidique.

**20.** Culture de cellules-hôtes, comprenant :
(a) un oncogène sous le contrôle transcriptionnel d'un promoteur hétérologue ;
(b) un gène codant pour un polypeptide désiré ; et
(c) un milieu de culture contenant le polypeptide désiré.

**21.** Culture de cellules suivant la revendication 20, dans laquelle le polypeptide désiré n'est pas nécessaire à la croissance ou la survie de la culture de cellules.

**Revendications pour l'Etat contractant suivant : ES**

**1.** Procédé comprenant :
(a) la transfection d'une cellule-hôte eucaryotique mère, rendue immortelle, avec un acide nucléique codant pour un polypeptide désiré et avec un oncogène ; et
(b) l'identification des transfectants de l'étape (a) qui produisent des quantités du polypeptide désiré par volume de milieu de culture qui sont supérieures à celles obtenues à partir de la cellule-hôte mère transfectée avec un acide nucléique codant pour le polypeptide désiré mais non avec l'oncogène.

**2.** Procédé suivant la revendication 1, dans lequel la cellule-hôte mère est transfectée avec l'acide nucléique codant pour le polypeptide désiré avant transfection de la cellule-hôte avec l'oncogène.

**3.** Procédé suivant la revendication 1 ou 2, dans lequel la cellule-hôte eucaryotique est une cellule de mammifère.

**4.** Procédé suivant la revendication 1, 2 ou 3, dans lequel le polypeptide est d'origine humaine.

**5.** Procédé suivant l'une quelconque des revendications 1 à 4, dans lequel le polypeptide est un activateur du plasminogène, l'inhibine, le TGF-$\beta$, l'activine, la prorelaxine, la superoxyde-dismutase, un facteur tissulaire, le facteur d'accélération de dégradation, un antigène viral ou une enképhalinase.

**6.** Procédé suivant la revendication 5, dans lequel le polypeptide est le t-PA.

**7.** Procédé suivant l'une quelconque des revendications 1 à 6, dans lequel la cellule-hôte est également transfectée avec un marqueur amplifiable.

**8.** Procédé suivant l'une quelconque des revendications 1 à 7, dans lequel l'oncogène est un acide nucléique viral.

**9.** Procédé suivant la revendication 8, dans lequel l'oncogène est l'oncogène v-src.

**10.** Procédé suivant l'une quelconque des revendications 1 à 9, dans lequel le polypeptide n'est pas nécessaire à la viabilité ou la croissance de la cellule-hôte mère.

**11.** Procédé suivant l'une quelconque des revendications 1 à 10, comprenant en outre la culture des transfectants de l'étape (b) jusqu'à ce que le polypeptide désiré s'accumule dans la culture, puis la séparation du polypeptide désiré de la culture.

**12.** Procédé suivant l'une quelconque des revendications 1 à 11, dans lequel le polypeptide est un polypeptide sécrété.

**13.** Procédé suivant l'une quelconque des revendications 1 à 12, dans lequel la cellule-hôte est également transfectée avec un acide nucléique codant pour un facteur de croissance polypeptidique.

20

**14.** Procédé suivant la revendication 13, dans lequel le facteur de croissance est l'insuline ou la proinsuline.

**15.** Procédé suivant l'une quelconque des revendications 1 à 14, dans lequel l'acide nucléique codant pour le polypeptide désiré n'est pas sous le contrôle transcriptionnel de ses séquences cellulaires naturelles de régulation de transcription.

**16.** Procédé suivant la revendication 15, dans lequel l'acide nucléique codant pour le polypeptide désiré est sous le contrôle d'un promoteur viral.

**17.** Procédé suivant la revendication 1, dans lequel le polypeptide est l'activateur du plasminogène tissulaire humain (t-PA).

**18.** Procédé suivant la revendication 17, dans lequel l'oncogène est l'oncogène v-src et la cellule-hôte est issue d'un mammifère ne consistant pas en un primate.

**19.** Procédé suivant la revendication 17 ou 18, dans lequel la cellule-hôte est également transfectée avec un acide nucléique codant pour un facteur de croissance polypeptidique.

FIG.1

FIG.2

F/G.3

FIG.4